# EUROPEAN PATENT APPLICATION

(11) **EP 0 841 056 A2**
(43) Date of publication of application: **13.05.1998**
(21) Application number: 97121041.4
(22) Date of filing: 21.02.1996
(51) Int. Cl.: A61H 39/00

(54) **Personal clothing with effects caused by weak electromagnetic waves**

(30) Priority: 01.03.1995 JP 42044/95
(62) Divisional of application: 96301138.2
(71) Applicant: Mineta, Hisashi, Shinjuku-ku, Tokyo-To (JP)
(72) Inventor: Mineta, Hisashi, Shinjuku-ku, Tokyo-To (JP)
(74) Representative: Spall, Christopher John

(57) **Abstract**

This invention induces a therapeutic effect such as "zen" by an article of clothing, by the formation generating far-infrared radiation (weak electromagnetic waves) at a portion corresponding to an abdominal region under the navel. It also prevents VDT-related disorders caused by weak electromagnetic waves by the insertion of salt, benzoic acid, or citric acid between a portion exposed to such waves and the human body. A therapeutic effect is obtained by temperature stimulus caused by such electromagnetic waves. VDT-related disorders can be prevented by reducing the stimulus caused by weak electromagnetic waves on the skin, by inserting salt, benzoic acid, or citric acid between a metal accessory and the body, or between a VDT and the body.

## Description

The present invention relates to an article of clothing that is designed to apply the natural feeling of the traditional medicine in East-Asia to induce a therapeutic effect such as "zen" using weak electromagnetic waves (far-infrared radiation) and to prevent disorders caused by weak electromagnetic waves.

In the prior art there have been several attempts to achieve a therapeutic effect or to prevent disorders by implementing certain devices on an item of clothing.

These include: a method of employing electrostatic induction by affixing metal pieces at the locations of meridian (so-called "acupuncture") points that are considered effective in the traditional medicine in East-Asia (see Japanese Utility Model Laid-Open Publication No. 63-143357 by the present inventor), a health or medical implement in accordance with that invention is provided with a non-magnetized and non-metal and narrow conductive substance having an electrical resistance that is intermediate between those of a conductive material and an insulating material (see Japanese Patent Laid-open Publication No. 6-125997 by the present inventor), a garment that is intended to increase the temperature of meridian points at afflicted portions of the human body by far-infrared radiation generated from a material that is made by a ceramic and an alloy thereof with germanium (trade name "seradi"), and clothing or garments that utilize metal to provide an electromagnetic shielding effect (see Japanese Utility Model Laid-Open Publication Nos. 61-64115 and 61-247477).

However, many of these prior-art articles of clothing that are designed to have a therapeutic effect or preventing disorders are harmful in that the direct stimulus thereof on the human body is too strong. In addition, there is no consideration of electrostatic induction in garments in which metal is utilized to provide an electromagnetic shielding effect, so they too are harmful to the human body.

The present invention was devised in the light of the above described problems with the prior art and has as an objective thereof the provision of an article of clothing that is intended to induce a therapeutic effect, by the formation of a subtle high-temperature portion at a portion of the garment corresponding to an abdominal region under the navel having internal organ cavity reflection and meridian points. This invention also provides an article of clothing that is intended to prevent damage to the health by the formation of a substance that obstructs induction by weak electromagnetic waves at a portion corresponding to a meridian point or a portion of skin of the human body.

The article of clothing in accordance with the present invention is formed to have a portion thereof that is at a portion corresponding to a meridian point of the body, or is of a form or material that obstructs unwanted stimulation.

When a person wears a garment in accordance with the present invention, a portion thereof comes into contact with a skin having meridian point of the wearer. This portion is formed in the cloth of the garment. A therapeutic effect is generated by a stimulus generated during that time, or by effects achieved by a subtle contact stimulus and an increase in temperature. Alternatively, damage to the health can be prevented by obstructing unwanted stimulus thereby.

Embodiments of the present invention will now be described with reference to the accompanying drawings, in which:

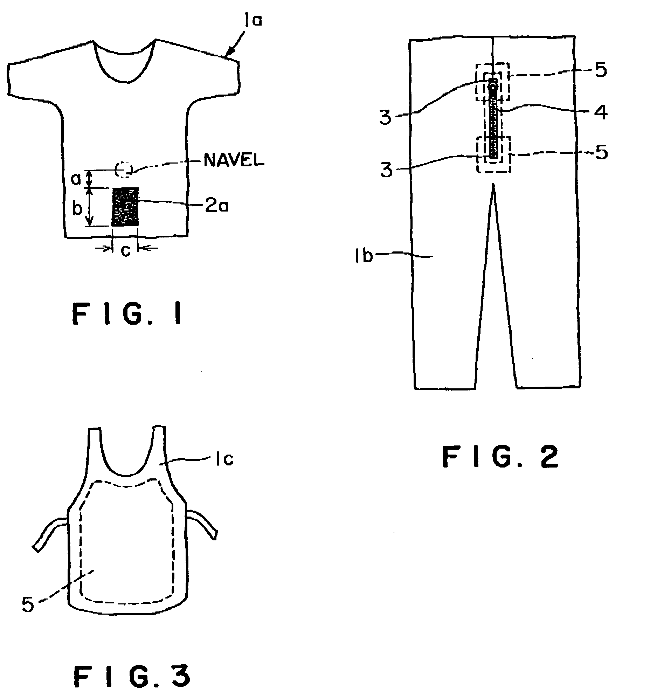

Fig. 1 is a schematic diagram of clothing that is an embodiment of articles of clothing (a tee-shirt) to which the present invention is applied;
Fig. 2 is a schematic view of slacks for preventing VDT-related disorders, which is a further embodiment of the article of clothing to which the present invention is applied, wherein a material covered with a non-permeable, non-metallic film formed as a film by means such as mixing salt, benzoic acid, or citric acid with an adhesive is affixed to an inner surface of the slacks between the body and metal portions of a mainly nylon zip fastener;
Fig. 3 is a schematic view of an apron for preventing VDT-related disorders, which is a still further embodiment of the article of clothing to which the present invention is applied, wherein a main part thereof is a material covered with a non-metallic film formed as a film by means such as mixing salt, benzoic acid, or citric acid with an adhesive.

To provide a basic knowledge for understanding the present invention, well known meridian (acupuncture) points of the human body will be briefly explained with respect to their positions on the basis of the disclosure of EP 0 729 738 A2 relating to the parent application.

The Tanzhong ("danchuu") point on the Conception Vessel meridian is on the chest;

The Mingmen ("meimon") and Changqiang ("choukyou") points on the Governor Vessel meridian is on the back;

The Sanyinjiao ("saninkou") point on the Spleen meridian is on the leg;

The Jiexi "kaikei" point on the Stomach meridian is on the leg; and

The Taichong "taishou" point on the Liver meridian is also on the leg.

Prior to the description of the present invention, the functions, i.e., stimuli to the human body and mental effects, of the meridian (acupuncture) points used for the East-Asian acupuncture will be described below (see EP 0 729 738 A2 relating to the parent European application of the present inventor). The phenomena occurring in relation to the meridian points can be confirmed but the reasons why the phenomena occur have been not fully clarified theoretically and scientifically. Therefore, only the phenomena will be referred to below.

Representative examples of some of the meridian points that are effective in the treatment of the human body are as follows. These are Tanzhong ("danchuu") on the Conception Vessel meridian, Mingmen ("meimon") and Changqiang ("choukyou") on the Governor Vessel meridian, Sanyinjiao ("saninkou") on the Spleen meridian and Jiexi ("kaikei") on the Stomach meridian, and Taichong ("taishou") on the Liver meridian (Here, all the notations of these meridian points are the simplified international notations established by the World Health organization (WHO), and the other notations in the brackets are the phonetic notations of the Japanese readings of the Chinese characters thereof.). The articles of clothing in accordance with the present invention use these meridian points (acupuncture points) as objects of treatment. Note that classification numbers for these meridian points have been laid down by WHO in such a manner that Tanzhong ("danchuu") is CV17, Mingmen ("meimon") is GV4, Changqiang ("choukyou") is GV1, Sanyinjiao ("saninkou") is Sp6, Jiexi ("kaikei") is S41, and Taichong ("taishou") is Liv3.

A first example disclosed in EP 0 729 738 A2 applied to swimming trunks is formed to have a thicker portion, fine protrusions, or a thinner portion at a portion thereof that corresponds to the Changqiang ("choukyou") point at the tip of the coccyx. When this portion comes into contact with the Changqiang ("choukyou") point of the wearer of these trunks, a therapeutic effect such as a raising or lowering of contact stimulus or an increase or decrease in the temperature of that portion is obtained. In particular, if fine protrusions are attached to the trunks, the contact stimulus thereof intensifies the stimulation of the Changqiang ("choukyou") point, which is used in treating serious illnesses. This augments the branch of the traditional medicine in East-Asia that acts to clear meridian points. If a subtly thicker portion is provided, the contact stimulus becomes weaker and the temperature at that meridian point increases This augments the traditional medicine in East-Asia with possibly serious illnesses, and clears the meridian point. If a subtly thinner portion is provided, not only is the contact stimulus lessened, but the temperature of the meridian point is reduced. This is used for lesser illnesses. This acts as a purgative for the traditional medicine in East-Asia by closing the meridian point.

The method of forming the thicker portion could basically be lacing. However, this portion could be directly attached by sewing to the predetermined location on the cloth of the trunks, or it could be made thicker than the cloth by incorporating it into the knitting thereof. Alternatively it could be affixed in the form of an adhesive plaster. In another possible method, cloth of the same thickness as that of the garment such as the trunks could be used to form a double- or triple-layer structure affixed to the inner side of the trunks. This would intensify the pressure on the meridian point to some extent, but increase the good therapeutic effect by further increasing the temperature-preserving effect.

A second example disclosed in the above publication, applied to a tee-shirt has a thicker portion on a shoulder portion thereof. Note that a tendency exists that a person with cold shoulders, first has a bad digestion, and then has a lumbago.

A third example disclosed in the above publication, applied to the front portion of a sleeveless undershirt is formed to have a thicker portion corresponding to the position of the Tanzhong ("danchuu") point.

A back portion of another tee-shirt is provided with a thicker portion corresponding to the Mingmen ("meimon") point.

A fourth example disclosed in the above publication, applied to a sock is designed to have a thicker portion corresponding to the Sanyinjiao ("saninkou") point. In this case, since there are individual differences in the position of the Sanyinjiao ("saninkou") point on the leg, the length of the thicker portion is extended somewhat along the Spleen meridian to which the Sanyinjiao ("saninkou") point belongs. Note also that this sock should also be marked in some suitable manner to enable the wearer to determine whether it is for the left foot or the right foot The Sanyinjiao ("saninkou") point is also known as the "onna-sanri" point, which is female's effective spot, and is effective in the treatment of ailments such as bad circulation in the feet and menstrual irregularities. This sock could also be formed to have a thicker portion or protrusions corresponding to the Jiexi ("kaikei") point on the Stomach meridian and the Taichong ("taishou") point on the Liver meridian. These points are markedly effective in recovery from mental fatigue and in ensuring spiritual stability.

Out of the garments configured as described above, if the stimulus of the swimming trunks is too strong it could lead to an adverse reaction and even actual harm (Changqiang is a sensitive meridian point). Therefore, wearing these trunks for only about 30 minutes, once a day, rather than for a long time, is conducive to health. This makes them suitable for wear as a "tee-back" swimming costume by someone of a delicate constitution.

Part of the garment could be removed by means such as cutting a hole or leaving open a portion thereof that corresponds to a meridian point of the human body or a portion of skin that has internal organ cavity reflection. Since this causes an open area to correspond to the meridian point or portion of skin, direct stimulus of that portion or the periphery thereof is removed and thus temperature-induced stimulus is reduced and also the humidity at that position is reduced so that a predetermined therapeutic effect is obtained. This embodiment is exemplified by the formation of an opened edge in underwear in the shape of a mini-skirt. This has the difficult-to-reverse advantage of a health method in which underpants are not worn, which is effective, hygienic, and a healthy custom.

The example in the above publication can also be applied to a garment, wherein portions under sleevebands of the garment that come into contact with the armpits are treated with an antibacterial deodorant treatment to prevent the propagation of bacteria that are the cause of smells, and also provide a strong contact stimulus by solid protrusions. It has long been verified that sweat is generated on the right side of the body if strong pressure is applied to the left armpit (by the activation of the auxiliary sympathetic nerves of the right side of the body). Such stimulus to left and right has effects such as sedating autonomous nerves, reducing the activity of the apocrine glands over the entire body (which would otherwise cause problems such as body odor), reducing halitosis, and promoting spiritual stability. This is effective when it is necessary to stimulate an activation of the sympathetic nerves by a stimulus such as a lowering of the temperature at only the armpit portions on either side. (Note that this is purgative on the traditional medicine in East-Asia.) Such effects could be obtained by an embodiment of an article of personal adornment that is thinner in armpit portions, or an article of clothing (underwear) that has cut-outs at armpit portions.

The present invention shown in Fig. 1 is embodied by affixing a non-metallic substance that generates far-infrared radiation (weak electromagnetic waves) to a location on the garment 1a that corresponds to the abdominal region under the navel 2a, to induce a therapeutic effect. For M-size clothing for males in Europe and America, the sizes of the location may be such that, as indicated in Fig. 1, the distance a from the center of the navel to the upper portion of the location is approximately 20 mm, the length b of the vertical side is approximately 80 mm, and the length c of the horizontal side is approximately 30 mm. Many ways are known of utilizing far-infrared radiation, but not as an example of warming the abdominal region under the navel and thus making it possible to activate the life forces. Health methods such as those that apply forces to the abdominal region under the navel (so-called "seika-tanden" which is a spot specified by the traditional medicine in East-Asia and the lowest tanden of the three tandens) do exist, including ones such as "zen", "kikou" and "yoga" that involve a matching with breathing. This is the simplest method without pain for the brain to attain a state of meditation. However, the healthy effects of this embodiment are increased by warming this region with a thick cloth or warming it by far-infrared radiation, with no accompanying pain. Note that no inverse effect is obtained by applying a comparatively strong stimulus to the abdominal region under the navel. In this case the clothing should be of a relaxing nature.

The article of clothing may also be configured of a material containing a substance that reduces the stimulus from weak electromagnetic waves to the meridian point or skin, such as salt, benzoic acid, or citric acid.

A more specific example of this embodiment is stated in Japanese Patent Laid-Open Publication No. 6-125997 by the present inventor. A health or medical implement in accordance with that invention is provided with a non-magnetized and non-metal and narrow conductive substance having an electrical resistance that is intermediate between those of a conductive material and an insulating material, as well as an insulating member that ensures that this conductive substance does not come into contact with the skin of the human body, arranged in such a manner as to correspond to the position of a meridian point used in the traditional medicine in East-Asia, or an infected part of the human body (a part of a meridian or skin), depending on the symptoms of illness. Note that a high-frequency voltage in the skin of the human body is not changed by weak electromagnetic waves of a comparatively low frequency of approximately 100 kHz or less. Furthermore, under the relatively low frequency of electromagnetic waves having approximately greater than 100 kHz, the voltage in the narrow skin increases due to secondary electrostatic induction caused on the akin. To the contrary, under the relatively higher frequency of electromagnetic waves, the voltage in the skin decreases. Thus, the so-called shielding effect can be provided. It is also well known that the electrical resistance at meridian points are lower than the other part of the human body. This configuration applies an extremely good form of narrow treatment to the affected part of the body by generating high-frequency electrostatic induction around the non-metal and narrow conductive substance, raising a high-frequency voltage in the skin by weak electromagnetic waves of a comparatively low frequency of approximately 100 kHz or more (lower than 1 MHz).

When an operator, young or old, or male or female, under a circumstance with a weak electromagnetic field, wears metal accessories (such as metal spectacle frames, pierced or clip-on ear-rings, necklaces, brooches, badges, wristwatches, bracelets, shoes or sandals with metal in the soles, underwear containing metal wires, metal zip-fasteners, tie pins, metal clothes fasteners, belt buckles, coins, prior-art aprons for shielding from electromagnetic waves, metal false teeth) that are not magnetized naturally by the weak electromagnetic waves that strike the bodies of such people as they work, the voltage of skin is dramatically increased by approximately 100 kHz or less, due to electrostatic induction of electromagnetic waves. Since these electromagnetic waves are alternating, the same effect would be obtained by metal in indirect skin contact. Stimulation by electromagnetic waves due to a rise in skin voltage at a meridian point, meridian, or a portion of skin that has internal organ cavity reflection, where such stimulation is not necessary, causes a breakdown in health (various ailments such as irritation, drowsiness, dizziness, headaches, deterioration of eyesight, stiff shoulders, lumbago, pain in the knees, loss of energy, dull appetite, constipation, high blood pressure, hair loss, impotence, sleeplessness, obesity, bad circulation, irregular periods, sterility, miscarriage, deterioration of immunity, failures of the auto-immune system such as atopic dermatitis and allergies, irregularities of the autonomous nervous system, neuroses, problems with the menopause, immorality, harassment, excessive computer gaming, and refusal to attend school). Therefore it is preferable that such metal accessories are not worn. It is further preferable that the effects of this other invention are exhibited to the full by avoiding the wearing of such metal accessories. In general, such phenomenon are hardly noticeable because they are masked by homeostasis, but it is necessary to be aware that the non-heating actions of electromagnetic waves are a cause of ill-health. Note that this point must also be considered in animal experiments measuring stress and the effects of electromagnetic waves. Detailed electromagnetic data of the human body are not generally known although there are data concerning the immunity, it being only known that the meridian (acupuncture) points of the human body have low electric resistance. Under these circumstances, measuring instruments such as measuring probes for human body are not available in the market.

Existing methods of preventing these VDT-related disorders include a nylon fastener at a portion of an article of clothing from which it is difficult to remove metal accessories, such as that shown in Fig. 2. However, such problems can be solved by inserting salt (such as natural salt in the main NaCl), benzoic acid, or citric acid between this metal portion 3 and the human body. A pair of slacks 1h that is another embodiment of the garment in accordance with this invention is fabricated as slacks for preventing such VDT-related disorders. A part 5 that is a material covered with a non-permeable, non-metallic film formed as a film by means such as mixing salt, benzoic acid, or citric acid with an adhesive is affixed to an inner surface of the slacks 1b between the body and metal portions 3 of a mainly nylon zip fastener. This reduces the stimulus of weak electromagnetic waves by metal accessories on the meridian points or skin. If VDT-related disorders occur after this problem of metal accessories has been solved, the stimulus of weak electromagnetic waves on the meridian points or skin can be further reduced by fixing salt (such as natural salt), benzoic acid, or citric acid to the front surface of the human body. An apron 1c for preventing VDT-related disorders, which is a further embodiment of this invention, comprises a part 5 that is formed of a material covered with a non-metallic film formed by means such as mixing salt, benzoic acid, or citric acid with an adhesive and applying it as a film thereto. Note that the action of salt, benzoic acid, or citric acid is a matter of personal experience and consumptive; theoretical analysis is still awaited. The effect of gait, benzoic acid and citric acid cannot be maintained semi-permanently for the above purpose, even if they are used in a sealed state, and is subject to deterioration after use for a short period. Therefore, it is required to make them exchangeable with a fresh one. In the case of the slacks shown in Fig. 2, it is appropriate that the part 5 is made exchangeable. In the case of an apron, it should bear an indication of the time limit until which the part 5 is effective.

Note also that it is inappropriate in the utilization of the effects of the present invention that garments that constrict the body should apply a too-large stimulus for a long time.

By using the locations of skin having meridian points to apply subtle and simple stimulus, or prevent harmful stimulus thereof, the present invention makes it possible to increase physical strength, regenerate life forces, activate the body's own power of healing, active auto-immune responses, provide spiritual stability, preserve health, promote circulation, and prevent and treat ailments such as headaches, stiff shoulders, menstrual pain, and bad circulation.

## Claims

1. An article of clothing comprising parts made of salt, benzoic acid, or citric acid in layer form and covered with a non-permeable film.

2. A non-metallic article of clothing inserted between a human body and a metal article of clothing, comprising parts made of salt, benzoic acid, or citric acid in layer form and covered with a non-permeable film.

3. An article of clothing wherein a non-metallic material for generating far-infrared radiation is attached to a portion thereof corresponding to an abdominal region under the navel.
